# EUROPEAN PATENT APPLICATION

(11) **EP 3 954 389 A1**
(43) Date of publication of application: **16.02.2022**
(21) Application number: 20759118.1
(22) Date of filing: 21.02.2020
(51) Int. Cl.: A61K 45/00, A61P 35/00, A61K 31/675

(54) **MTOR INHIBITOR**

(30) Priority: 22.02.2019 JP 2019030944
(71) Applicant: Murata, Kazumoto, Tochigi 329-0498 (JP)
(72) Inventor: MIZOKAMI, Masashi, Ichikawa-shi, Chiba 272-8516 (JP); MURATA, Kazumoto, Shimotsuke-shi, Tochigi 329-0498 (JP)
(74) Representative: Hoffmann Eitle
(86) International application number: PCT/JP2020/007172
(87) International publication number: WO 2020/171225

(57) **Abstract**

The present invention relates to an mTOR inhibitor containing adefovir or tenofovir or a prodrug thereof, or a pharmaceutically acceptable salt thereof.

## Description

### Technical Field

The present invention relates to an mTOR inhibitor.

### Background Art

Adefovir (ADV) and tenofovir (TDF) are known as drugs used in the treatment of hepatitis B virus (HBV) infectious disease.

Non-Patent Document 1 discloses that a serum IFN-λ3 value shows a high value only in hepatitis B patients taking ADV or TDF, and ADV or TDF induces IFN-λ3 in an intestinal cell by using an in vitro system. Furthermore, it is disclosed that the induced IFN-λ3 induces IFN-stimulated genes (ISG) in a hepatocyte, and an HBsAg production suppressing effect is shown.

Meanwhile, in some patients with hepatitis B virus infectious disease, inflammation occurs in the liver due to the enhancement of the autoimmune system with respect to the liver infected with hepatitis B virus, and due to chronic inflammation, the disease progresses to chronic hepatitis B and liver cirrhosis, and eventually liver cancer develops.

Non-Patent Document 2 shows the results of a cohort study conducted in the Republic of Korea and relating to the risk of hepatocellular carcinoma in patients treated for chronic hepatitis B.

### Citation List

### Non-Patent Document

Non-Patent Document 1: Gut. 2018 67 (2): 362-371
Non-Patent Document 2: JAMA Oncol. 2019 5 (1): 30-36

### Summary

### Technical Problem

Although there is a possibility that the development of liver cancer can be prevented from happening by the treatment of hepatitis B virus infectious disease with ADV and TDF, it has not been known so far whether ADV and TDF have a direct antitumor effect.

An object to be achieved by the present invention is to provide a new compound having an antitumor effect.

### Solution to Problem

The inventors of the present invention have conducted diligent studies to achieve the above-mentioned object, have found that ADV and TDF have an antitumor effect on the basis of that ADV and TDF have an mTOR inhibitory effect and an Akt phosphorylation inhibitory effect, and therefore have completed the present invention.

That is, the present invention is as follows.
(1) An mTOR inhibitor containing: adefovir or tenofovir or a prodrug thereof, or a pharmaceutically acceptable salt thereof.
(2) An Akt phosphorylation inhibitor containing: adefovir or tenofovir or a prodrug thereof, or a pharmaceutically acceptable salt thereof.
(3) An antitumor agent containing: adefovir or tenofovir or a prodrug thereof, or a pharmaceutically acceptable salt thereof.
(4) The antitumor agent according to (3), in which a tumor is a tumor not caused by hepatitis.
(5) The antitumor agent according to (4), in which the hepatitis is hepatitis B.
(6) The antitumor agent according to any one of (3) to (5), in which the antitumor agent is administered to a patient not infected with a hepatitis virus.
(7) The antitumor agent according to any one of (3) to (6), in which the antitumor agent is used in combination with another antitumor agent.
(8) An mTOR inhibitor and/or an Akt phosphorylation inhibitor which are for treating a tumor and/or cancer, in which the mTOR inhibitor and/or the Akt phosphorylation inhibitor are used by being administered to preferably a patient and more preferably a human patient, and contain a treatment effective amount/a therapeutically effective amount of adefovir or tenofovir or a prodrug thereof, or a pharmaceutically acceptable salt thereof.

The tumor and/or cancer is preferably a tumor and/or cancer not caused by hepatitis, and may be a tumor and/or cancer not caused by hepatitis B. The patient or human patient may be for administration to a patient or human patient not infected with a hepatitis virus. The mTOR inhibitor or the Akt phosphorylation inhibitor may be used in combination with another antitumor agent.
(9) An antitumor agent for treating a tumor and/or cancer by inhibiting mTOR and/or inhibiting Akt phosphorylation, in which the antitumor agent for treating a tumor and/or cancer is used by being administered to preferably a patient and more preferably a human patient, and contains a treatment effective amount/a therapeutically effective amount of adefovir or tenofovir or a prodrug thereof, or a pharmaceutically acceptable salt thereof.

The tumor and/or cancer is preferably a tumor and/or cancer not caused by hepatitis, and may be a tumor and/or cancer not caused by hepatitis B. The patient or human patient may be for administration to a patient or human patient not infected with a hepatitis virus. The antitumor agent may be used in combination with another antitumor agent.

(10) An mTOR inhibitor and/or an Akt phosphorylation inhibitor containing adefovir or tenofovir or a prodrug thereof, or a pharmaceutically acceptable salt thereof may be used in a method of treating a tumor and/or cancer by administering a treatment effective amount/a therapeutically effective amount thereof to a subject in need thereof, preferably a patient, and more preferably a human patient. Furthermore, adefovir or tenofovir or a prodrug thereof, or a pharmaceutically acceptable salt thereof may be used in a method of treating a tumor and/or cancer by administering a treatment effective amount/a therapeutically effective amount thereof to a subject in need thereof, preferably a patient, and more preferably a human patient, may be used in a method of inhibiting mTOR and/or inhibiting Akt phosphorylation, or may be used in a method of treating a tumor and/or cancer by inhibiting mTOR and/or inhibiting Akt phosphorylation.

The tumor and/or cancer is preferably a tumor and/or cancer not caused by hepatitis, and may be a tumor and/or cancer not caused by hepatitis B. The patient or human patient may be for administration to a patient or human patient not infected with a hepatitis virus. The mTOR inhibitor or the Akt phosphorylation inhibitor may be used in combination with another antitumor agent.
(11) Adefovir or tenofovir or a prodrug thereof, or a pharmaceutically acceptable salt thereof for inhibiting mTOR and/or inhibiting Akt phosphorylation;
adefovir or tenofovir or a prodrug thereof, or a pharmaceutically acceptable salt thereof for treating a tumor and/or cancer;
adefovir or tenofovir or a prodrug thereof, or a pharmaceutically acceptable salt thereof for treating a tumor and/or cancer by inhibiting mTOR and/or inhibiting Akt phosphorylation;
use for producing a pharmaceutical formulation and/or pharmaceutical composition comprising adefovir or tenofovir or a prodrug thereof, or a pharmaceutically acceptable salt thereof for treating a tumor and/or cancer, for inhibiting mTOR and/or inhibiting Akt phosphorylation, or for treating a tumor and/or cancer by inhibiting mTOR and/or inhibiting Akt phosphorylation; and
use of adefovir or tenofovir or a prodrug thereof, or a pharmaceutically acceptable salt thereof for inhibiting mTOR and/or inhibiting Akt phosphorylation, for treating a tumor and/or cancer, or for treating a tumor and/or cancer by inhibiting mTOR and/or inhibiting Akt phosphorylation.

A treatment effective amount/a therapeutically effective amount of adefovir or tenofovir or a prodrug thereof, or a pharmaceutically acceptable salt thereof may be administered to a subject in need thereof, preferably a patient, and more preferably a human patient.

The tumor and/or cancer is preferably a tumor and/or cancer not caused by hepatitis, and may be a tumor and/or cancer not caused by hepatitis B. The patient or human patient may be for administration to a patient or human patient not infected with a hepatitis virus. Another antitumor agent.may be used in combination.

### Advantageous Effects of Invention

According to the present invention, it is possible to provide a new compound having an antitumor effect.

### Brief Description of Drawings

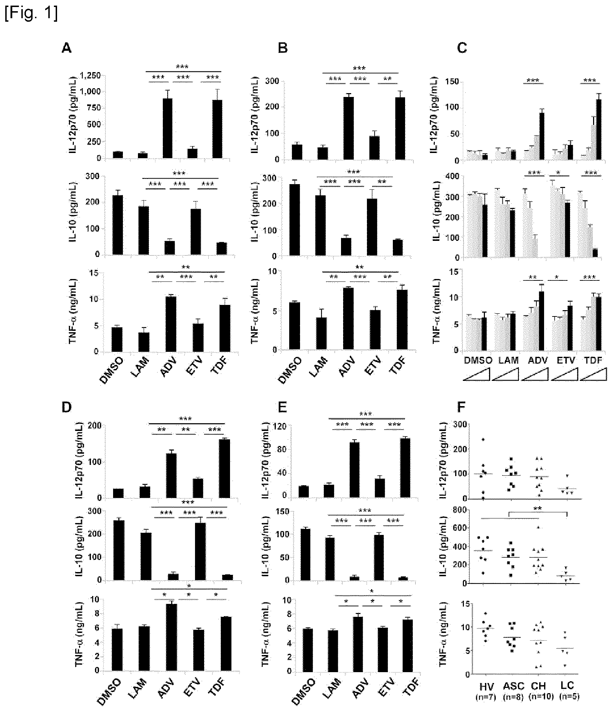

Fig. 1 shows the test results of the regulation of a cytokine profile in a peripheral blood mononuclear cell (PBMC). Fig. 1(A) shows a representative result among three healthy volunteers (HV). Fig. 1(B) shows the results of enzyme-linked immunosorbent assay (ELISA) of HV of seven persons. Fig. 1(C) shows that, in Examples, ADV indicates adefovir pivoxil and TDF indicates tenofovir disoproxil fumarate. Dose-dependent production of interleukin (IL)-12p70, IL-10, and tumor necrosis (TNF)-α by ADV and TDF was observed (1:3 dilution). The number n is at least 3. Fig. 1(D) shows the results of using a PBMC from a chronic hepatitis B patient (CHB; 44-year-old man, HBeAg+, genotype A). Fig. 1(E) shows the results of using a PBMC from a liver cirrhosis (LC) B patient (68-year-old woman, HBeAg-, genotype C). Fig. 1(F) shows the results of treatment with 500 nM of ADV using a PBMC from HV (n = 7), an asymptomatic carrier (n = 8), a CH patient (n = 10), and an LC patient (n = 5) (**: p < 0.01 and ***: p < 0.001).

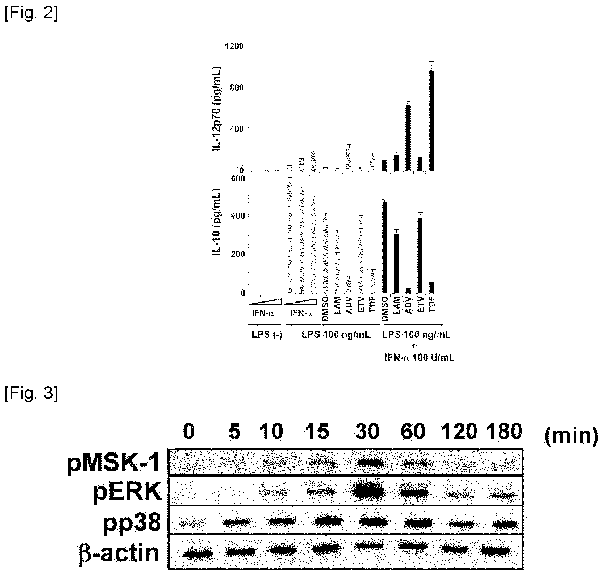

Fig. 2 shows the test results of synergistic induction of LPS-stimulated IL-12p70 production by using in a combination with interferon (IFN)-α. In the case of interferon (IFN)-α alone, 10, 100, and 1000 U/mL were used.
Fig. 3 shows the passage of time of phosphorylation of mitogen-activated protein kinase (MAPK)-related protein after lipopolysaccharide (LPS) stimulation. A CD14+ monocyte positively selected using magnetic-activated cell sorting (MACS) was stimulated with 100 ng/mL of LPS to recover the cell at each time point. The results of performing western blot analysis using phosphorylated-pediatric musculoskeletal (pMSK)-1, pERK (extracellular signal-regulated kinase), and pp38 as primary antibodies are shown. Phosphorylation of these proteins reached a peak 30 minutes after LPS stimulation.

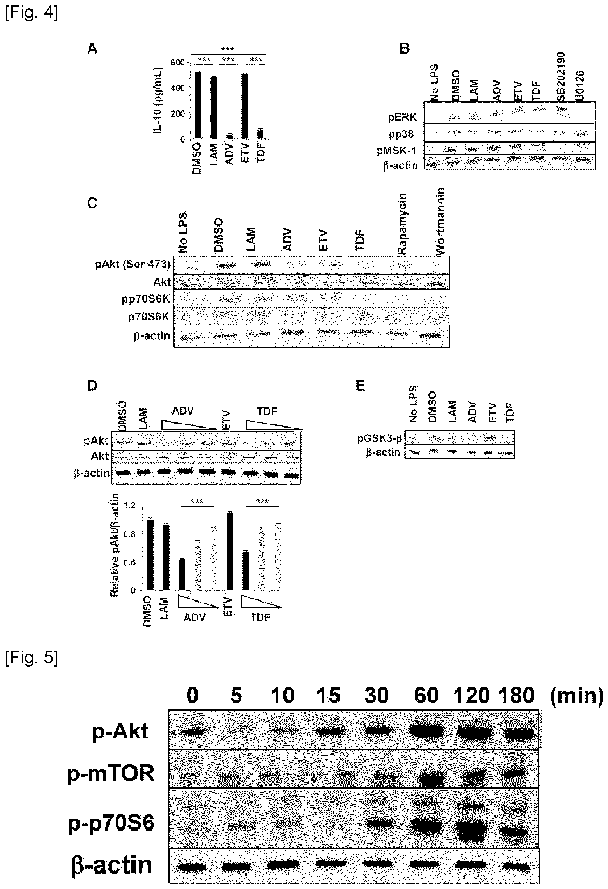

Fig. 4 shows the test results of inhibition of an mTOR pathway. In Fig. 4(A), a CD14+ monocyte was positively selected by magnetic-activated cell sorting (MACS), pretreated with a nucleic acid analog for 2 hours, and then stimulated with 100 ng/mL of lipopolysaccharide (LPS). The results of measuring IL-10 in the supernatant by enzyme-linked immunosorbent assay (ELISA) are shown. The degree of purity of the CD14+ monocyte was > 95%. Fig. 4(B) shows the results of performing western blot analysis by treating the CD14+ monocyte with the nucleic acid analog, 10 µM of SB202190, or 10 µM of U0126 for 2 hours, and subsequently performing stimulation with 100 ng/mL of LPS to recover the cell 30 minutes after LPS stimulation. In Fig. 4(C), the CD14+ monocyte was treated with 100 nM of the nucleic acid analog. Rapamycin or 5 µM of wortmannin was stimulated with 100 ng/mL of LPS for consecutive 2 hours. The results of performing western blot analysis by collecting the cell 2 hours after LPS stimulation are shown. In Fig. 4(D), the CD14+ monocyte was treated with the nucleic acid analog and subsequently stimulated with 100 ng/mL of LPS (1:3 dilution). In Fig. 4(E), the CD14+ monocyte was treated with the nucleic acid analog for 2 hours and subsequently stimulated with 100 ng/mL of LPS. The results of performing western blot analysis (phosphorylated glycogen synthase kinase [pGSK]-3β) by recovering the cell 2 hours after LPS stimulation are shown.
Fig. 5 shows the passage of time of phosphorylation of a mammalian target of rapamycin (mTOR)-related protein after lipopolysaccharide (LPS) stimulation. A CD14+ monocyte positively selected using magnetic-activated cell sorting (MACS) was stimulated with 100 ng/mL of LPS to recover the cell at each time point. The results of performing western blot analysis using pAkt1, pmTOR, and pp70S6K as primary antibodies are shown. Phosphorylation of these proteins reached a peak 2 hours after LPS stimulation.

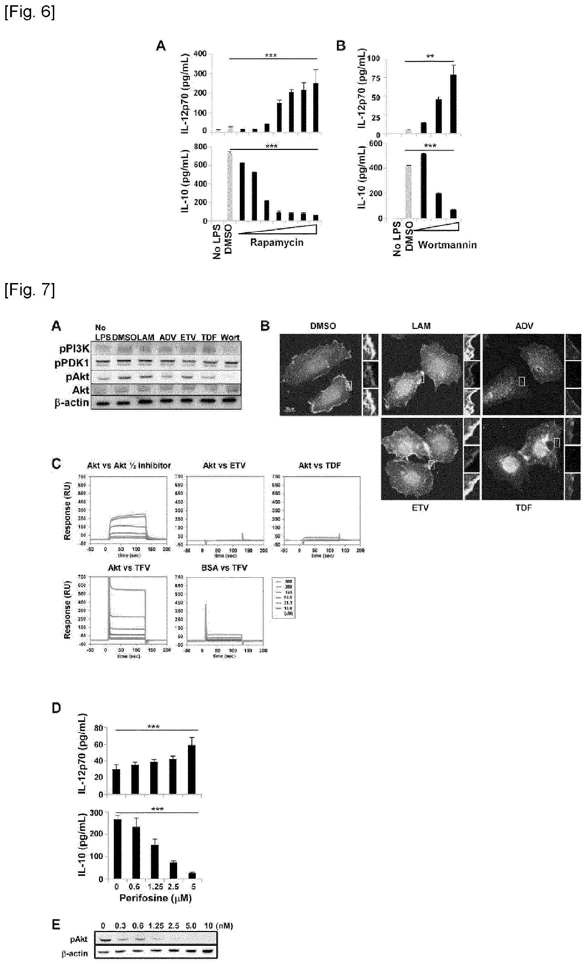

Fig. 6 shows the test results of cytokine regulation by an mTOR inhibitor.
Fig. 7 shows the test results of inhibition of the transition of Akt to the cell membrane. In Fig. 7(A), a CD14+ monocyte was pretreated with a nucleic acid analog or 5 µM of wortmannin (Wort) for 2 hours and thereafter stimulated with 100 ng/mL of LPS. The cell was recovered. The results of performing western blot analysis for phosphorylation of Akt upstream proteins (phosphatidylinositol-3 kinase [PI3K] and phosphoinositide-dependent kinase 1 [PDK1]) 2 hours after LPS stimulation are shown. Fig. 7(B) shows the results of treating HT-1080 cell stably expressing Akt2PH-EGFP with the nucleic acid analog for 2 hours, subsequently fixing, and staining with anti-phospho-Akt (Ser 473 and DAPI). The transposition (green) of Akt to the cell membrane and phosphorylation (red) of Akt were inhibited by ADV or TDF. Each inserted drawing shows a magnified image of the plasma membrane (Akt translocation; top, Akt phosphorylation; middle, confluence; bottom). Fig. 7(C) shows the results of testing the interaction between Akt and the nucleic acid analog using a surface plasmon resonance (SPR) biosensor. An Akt inhibitor 2 (Akt-1/2) and bovine serum albumin (BSA) were respectively used as a positive control and a negative control. In Fig. 7(D), a PBMC was pretreated with perifosine at various concentrations for 2 hours and subsequently stimulated with 100 ng/mL of LPS. The supernatant was measured by enzyme-linked immunosorbent assay (ELISA) 24 hours after LPS stimulation. Fig. 7(E) shows the results of performing western blot analysis after treating the PBMC with perifosine at various concentrations and thereafter recovering the cell 2 hours after LPS stimulation.

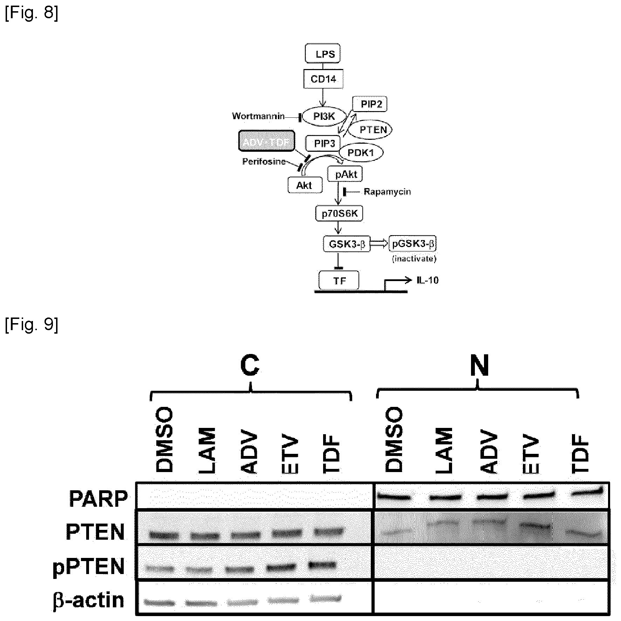

Fig. 8 shows a schematic diagram of the regulatory mechanism of LPS-mediated IL-10 production by ADV and TDF. The cellular metabolites of ADV and TDF bind to Akt protein, inhibit the translocation of Akt to the cell membrane, and thereafter inhibit phosphorylation thereof during LPS administration to inhibit LPS-mediated IL-10 production. TF represents a transcription factor.
Fig. 9 shows that a phosphatase and tensin homologue (PTEN) did not contribute to the inhibition of LPS-mediated IL-10 by ADV and TDF. A CD14+ monocyte positively selected by magnetic-activated cell sorting (MACS) was pretreated with a nucleic acid analog for 2 hours, and the cell was recovered without stimulation with LPS. The results obtained by further fractionating the cell into a cytosol fraction and a nuclear fraction, and performing western blot analysis on each of the obtained fractions are shown. Poly-ADP-ribose polymerase (PARP) is the internal control of a nuclear protein, and β-actin is the internal control of the cytosol fraction. The number n is at least 3.

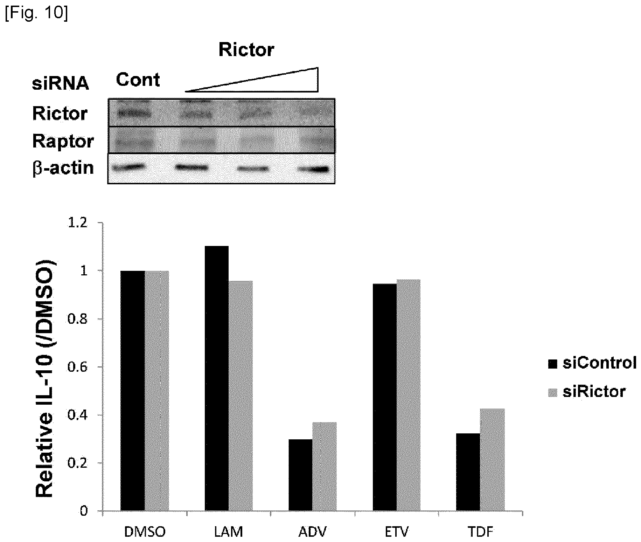

Fig. 10 shows that a mammalian target of rapamycin complex 2 (mTORC2) did not contribute to the inhibition of IL-10 by ADV and TDF. A CD14+ monocyte positively selected by magnetic-activated cell sorting (MACS) was transfected with control siRNA and rictor using a transfection kit. The results of confirming the expression levels of rictor, raptor, and β-actin 48 hours after transfection are shown. The results obtained by subsequently pretreating the cell with a nucleic acid analog for 2 hours, subsequently stimulating with 100 ng/mL of LPS, and measuring IL-10 in the supernatant by enzyme-linked immunosorbent assay (ELISA) 24 hours after LPS stimulation are shown.

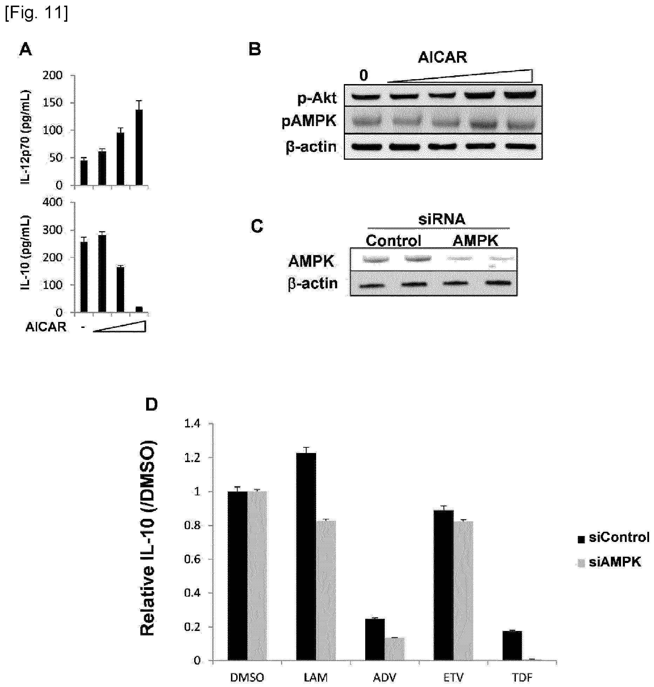

Fig. 11 shows that a 5'-AMP-activated protein kinase (AMPK) did not contribute to the inhibition of IL-10 by ADV and TDF. In Fig. 11(A), a PBMC was pretreated with 5-aminoimidazole-4-carboxamide-1-β-D-ribofuranoside (AICAR) at various concentrations for 2 hours and subsequently stimulated with 100 ng/mL of LPS. The results of analysis of IL-12p70 and IL-10 in the supernatant using enzyme-linked immunosorbent assay (ELISA) 24 hours after LPS stimulation are shown. In Fig. 11(B), a CD14+ monocyte positively selected using magnetic-activated cell sorting (MACS) was pretreated with AICAR at various concentrations for 2 hours and stimulated with 100 ng/mL of LPS. The results of performing western blot analysis by recovering the cell 2 hours after LPS stimulation are shown. In Fig. 11(C), a CD14+ monocyte positively selected using MACS was transfected with control siRNA and AMPK using a Nucleofector kit. The results of performing western blot analysis by recovering the cell 48 hours after transfection are shown. In Fig. 11(D), a CD14+ monocyte transfected with control siRNA and AMPK was pretreated with nucleic acid analog for 2 hours and subsequently stimulated with LPS for 24 hours. The results of measuring IL-10 in the supernatant by enzyme-linked immunosorbent assay (ELISA) are shown.

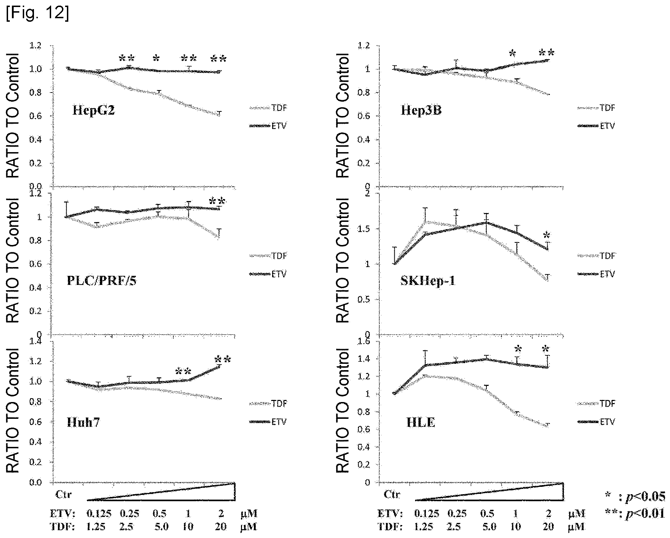

Fig. 12 shows the antitumor effect of entecavir (ETV) or TDF in various liver cancer cell lines.

### Description of Embodiments

A mammalian target of rapamycin (mTOR) inhibitor of the present invention contains adefovir or tenofovir or a prodrug thereof, or a pharmaceutically acceptable salt thereof.

Adefovir is a compound represented by Formula (I), and hereinafter, adefovir or a prodrug of adefovir, or a pharmaceutically acceptable salt thereof may be generically referred to as "adefovir and the like" in the present specification.

### Formula (I):

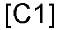

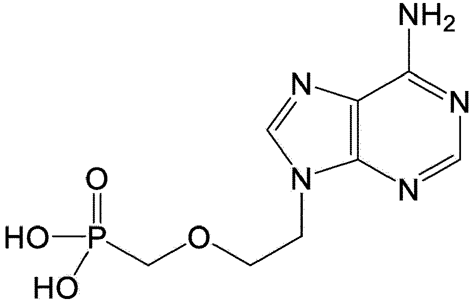

Tenofovir is a compound represented by Formula (II), and hereinafter, tenofovir or a prodrug of tenofovir, or a pharmaceutically acceptable salt thereof may be generically referred to as "tenofovir and the like" in the present specification.

### Formula (II):

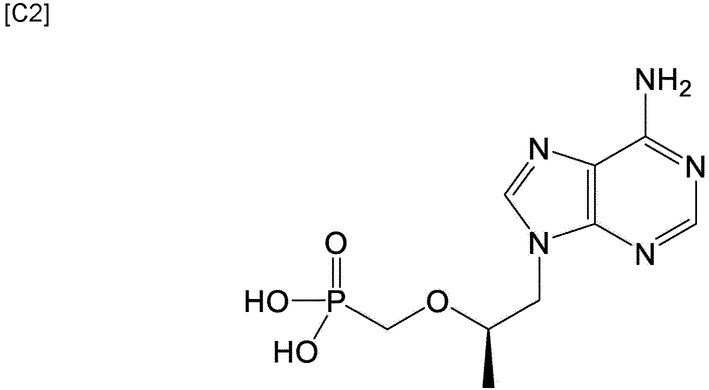

As the prodrug of adefovir and tenofovir, a prodrug known as a prodrug with a phosphoric acid group can be used, and a prodrug known as a nucleic acid analog prodrug is preferable.

The prodrug is not particularly limited, and examples thereof include a prodrug in which the phosphoric acid group is substituted with a pivoxil group, a disoproxil group, an alafenamide group, or the like.

Examples of the salt of adefovir and tenofovir or the salt of the prodrug of adefovir and tenofovir include alkali metal salts, alkaline-earth metal salts, ammonium salts, amino acid salts, organic acid salts, and inorganic acid salts.

**The** mTOR inhibitor of the present invention preferably contains adefovir and the like and/or tenofovir and the like as active ingredients.

The adefovir and the like and/or tenofovir and the like are not particularly limited, but for example, it is possible to suitably use adefovir pivoxil (also referred to as adefovir dipivoxil), tenofovir alafenamide fumarate, and tenofovir disoproxil fumarate.

In the present invention, the phrase "as an active ingredient" means that the adefovir and the like and/or tenofovir and the like are contained as main active component, and as long as adefovir and the like and/or tenofovir and the like are contained as medicinal ingredients (ingredients exerting a pharmacological effect), the content thereof is not particularly limited.

In the present invention, adefovir and the like and tenofovir and the like may be in the form of a hydrate or may be in the form of a solvate.

Furthermore, adefovir and the like and tenofovir and the like may be isotope-labeled. The isotope is not particularly limited, and examples thereof include 33P, 32P, 13C, and 2H (D), which are stable isotopes of a phosphorus atom, a carbon atom, and a hydrogen atom.

Because mTOR signaling is commonly activated in a tumor cell, the mTOR inhibitor of the present invention exhibits the antitumor effect by an mTOR inhibitory action.

In the present invention, an Akt phosphorylation inhibitor is provided, and the Akt phosphorylation inhibitor of the present invention contains adefovir or tenofovir or a prodrug thereof, or a pharmaceutically acceptable salt thereof.

Akt is a PI3K-dependently activated kinase and regulates downstream signaling such as Bad and GSK-3 to induce a mammalian target of rapamycin (mTOR). Accordingly, since the induction of mTOR is suppressed by the Akt phosphorylation inhibitor of the present invention, the Akt phosphorylation inhibitor of the present invention exhibits an antitumor effect.

In the present invention, the mTOR inhibitor or the Akt phosphorylation inhibitor can suppress the growth of a cancer cell and a sarcoma cell and induce cell death (apoptosis) by inhibiting a PI3K/Akt/mTOR pathway, and therefore, the mTOR inhibitor or the Akt phosphorylation inhibitor can be used as an antitumor agent.

An antitumor agent of the present invention contains adefovir or tenofovir or a prodrug thereof, or a pharmaceutically acceptable salt thereof.

A tumor for which the antitumor agent in the present invention is used is not particularly limited, and examples thereof include a hematopoietic tumor and a solid cancer.

Examples of the hematopoietic tumor include acute leukemia, chronic lymphocytic leukemia, chronic myeloid leukemia, polycythemia vera, malignant lymphoma, and myeloma.

Examples of the solid cancer include brain tumor, head and neck cancer, esophageal cancer, thyroid cancer, small cell lung cancer, non-small cell lung cancer, breast cancer, stomach cancer, gallbladder and bile duct cancer, liver cancer, pancreatic cancer, colon cancer, rectal cancer, ovarian cancer, chorionic epithelial cancer, uterine body cancer, uterine cervix cancer, urothelial cancer, renal cell cancer, prostate cancer, testicular tumor, Wilms tumor, malignant melanoma, neuroblastoma, osteogenic sarcoma, Ewing's sarcoma, and soft tissue sarcoma.

The antitumor agent of the present invention is used for the purpose of preventing and/or treating cancer, but is suitably used for cancer treatment.

Among these, the antitumor agent exhibits the effect of shrinking or eliminating a tumor or suppressing the growth of a tumor to below a certain level.

Unlike a drug that can be used for cancer prevention by treating the hepatitis B virus (HBV) infectious disease, the antitumor agent of the present invention suitably exhibits a direct antitumor effect by an mTOR inhibitory action or an Akt phosphorylation inhibitory action, and can be used as an antitumor agent with respect to a tumor not caused by hepatitis.

The hepatitis is not particularly limited but is preferably hepatitis B for which adefovir and/or tenofovir is used.

The antitumor agent of the present invention can be used as an antitumor agent with respect to tumors not caused by hepatitis, preferably not caused by hepatitis B. The tumors not caused by hepatitis, preferably not caused by hepatitis B, may be the above-mentioned hematopoietic tumor or solid cancer, but the antitumor agent may be used as an antitumor agent for liver cancer not caused by hepatitis, preferably not caused by hepatitis B.

The mTOR inhibitor, the Akt phosphorylation inhibitor, and the antitumor agent of the present invention (hereinafter, may be referred to as "antitumor agent and the like") are used as a drug, a pharmaceutical formulation, and/or a pharmaceutical composition, and can be administered orally or parenterally.

For oral administration, it is possible to use known administration forms such as tablets, capsules, coated tablets, troches, and liquid medications such as solutions or suspensions.

Examples of parenteral administration include intravenous, intramuscular, or subcutaneous administration by injection; transmucosal administration through the nasal cavity, oral cavity, and the like using powders, drops, sprays, aerosols, or the like; rectal administration using creams, suppositories, or the like; and transdermal administration using patches, liniments, gels, or the like.

The administration route is preferably oral administration or intravenous administration by injection.

In the present invention, the antitumor agent and the like may be used in combination with another antitumor agent.

The other antitumor agent can be appropriately selected depending on a target tumor.

The antitumor agent and the like in the present invention preferably further regulate a lipopolysaccharide (LPS)-mediated cytokine profile in a peripheral blood mononuclear cell (PBMC). Specifically, the antitumor agent and the like in the present invention inhibit LPS-induced interleukin (IL)-10 production to mutually induce LPS-mediated IL-12p70 and tumor necrosis factor (TNF)-α expression. Furthermore, the combination of IFN-α and the tumor agent and the like synergistically enhances LPS-mediated IL-12p70 production in a PBMC.

Downregulation of IL-10, which suppresses an excessive immune response, is expected to restore the functions of a T cell and a NK cell. Accordingly, the antitumor agent and the like in the present invention is preferably used in combination with another antitumor agent because the antitumor agent and the like enhances the antitumor activity of the other antitumor agent by not only exhibiting the mTOR inhibitory activity and/or the Akt phosphorylation inhibitory activity but also functioning as an IL-10 production inhibitor.

The antitumor agent and the like of the present invention may be the mTOR inhibitor or the Akt phosphorylation inhibitor, or the IL-10 production inhibitor.

The antitumor agent and the like of the present invention may be the mTOR inhibitor and the IL-10 production inhibitor, may be the Akt phosphorylation inhibitor and the IL-10 production inhibitor, or may be the mTOR inhibitor, the Akt phosphorylation inhibitor, and the IL-10 production inhibitor.

Furthermore, the antitumor agent and the like of the present invention may be an IL-12p70 production enhancer, may be a TNF-α production enhancer, may be an IL-12p70 expression inducer, or may be a TNF-α expression inducer.

The antitumor agent and the like of the present invention may be the mTOR inhibitor and the IL-12p70 production enhancer and/or the TNF-α production enhancer, may be the Akt phosphorylation inhibitor and the IL-12p70 production enhancer and/or the TNF-α production enhancer, or may be the mTOR inhibitor, the Akt phosphorylation inhibitor, and the IL-12p70 production enhancer and/or the TNF-α production enhancer. The antitumor agent and the like of the present invention may be the mTOR inhibitor and the IL-12p70 expression inducer and/or the TNF-α expression inducer, may be the Akt phosphorylation inhibitor and the IL-12p70 expression inducer and/or the TNF-α expression inducer, or may be the mTOR inhibitor, the Akt phosphorylation inhibitor, and the IL-12p70 expression inducer and/or the TNF-α expression inducer. Furthermore, the antitumor agent and the like of the present invention may be the IL-10 production inhibitor.

When the antitumor agent and the like are used as a pharmaceutical formulation and/or a pharmaceutical composition, in addition to the antitumor agent and the like and other tumor agents, an appropriate pharmaceutically acceptable carrier well known to those skilled in the art may be appropriately included depending on an administration form and the like.

The pharmaceutically acceptable carrier is not particularly limited, but examples thereof include antioxidants, stabilizers, preservatives, flavoring agents, colorants, dissolving agents, solubilizing agents, surfactants, emulsifiers, defoamers, viscosity adjusters, gelling agents, absorption accelerating agents, dispersants, excipients, and pH adjusters.

In the present invention, the dose and administration plan of the adefovir and the like and/or tenofovir and the like as the antitumor agent and the like may be adjusted depending on a required amount, treatment method, disease, or degree of necessity for each individual treatment target.

The dose can be specifically determined according to age, body weight, general health condition, gender, diet, administration time, administration method, excretion rate, drug combination, medical condition of a patient, and the like, and may be determined in consideration of other factors.

As the antitumor agent and the like of the present invention, the adefovir and the like and/or tenofovir and the like are preferably contained in an amount effective in exerting an antitumor effect.

The daily dose of the adefovir and the like and/or tenofovir and the like varies depending on a condition and body weight of a patient, type of compound, administration route, and the like, but in the case of parenteral administration, the daily dose is usually about 0.01 to 1000 mg/person/day, preferably 0.1 to 500 mg/person/day, and in the case of oral administration, the daily dose is usually about 0.01 to 500 mg/person/day, preferably 0.1 to 100 mg/person/day.

In the present invention, a method of preventing or treating cancer which includes administering the antitumor agent and the like to a patient in need thereof may be provided.

The patient in need thereof is not particularly limited, but is a mammal, preferably a human.

It is preferable that the antitumor agent and the like in the present invention be used for administration to a patient not infected with hepatitis virus as a patient to which administration is performed.

As the antitumor agent and the like, the adefovir and the like and/or tenofovir and the like are preferably administered in a treatment effective amount.

In the present invention, since the adefovir and the like and/or tenofovir and the like have an antitumor action, they may be used for prevention of cancer by administering them before the development of cancer or may be used for treatment of cancer.

### Examples

Hereinafter, the present invention will be more specifically described with reference to examples, but the present invention is not limited to the following examples. Furthermore, various changes can be made without departing from the technical idea of the present invention.

Lamivudine purchased from Sigma-Aldrich (St Louis, Missouri, USA) and other nucleic acid analog purchased from Adooq Bioscience (Irvine, CA, USA) were used. For phosphorylated primary antibodies (ERK, p38 (Thr180/Thy182), MSK-1 (Thr581), Akt (Ser 473), p70S6K (Thr389), 4E-BP1 (Thr37/Thr46), c-fos (Ser32), GSK-3β (Ser9), PDK1 (Ser241), PTEN (Ser380/Thr382/383), Akt, PARP, PTEN, and b-actin, those purchased from Cell Signaling Technology (Danvers, MA, USA) were used. For a phosphorylated primary antibody of PIK3R1/PIK3R3, one purchased from Thermo scientific (Rockford, IL, USA) was used. For recombinant proteins of a monoclonal antibody, IL-10, IL-12, and TNF-a, those purchased from R&D systems (Minneapolis, MN, USA) were used.

### <Separation of various lymphocyte cells>

A peripheral blood mononuclear cell (PBMC) was separated from blood collected from a healthy volunteer (HV) and a chronic hepatitis B patient by a concentration gradient method. CD3+ T cell, CD19+ B cell, CD56+ NK cell, and CD14+ monocyte were respectively separated using magnetic antibodies.

### <Cell culture>

PBMC or CD14+ monocyte was cultured for 2 hours in an RPMI solution containing 10% bovine serum and a nucleic acid analog formulation (NUC), rapamycin, wortmannin, and perifosine at various concentrations, and thereafter stimulated with 100 ng/mL of lipopolysaccharide (LPS) to collect a supernatant or a cell at the indicated time. Unless otherwise specified, for the concentrations of nucleic acid analogs used, 50 mM, 500 nM, 500 nM, and 5 mM were respectively used for lamivudine (LAM), adefovir (ADV), entecavir (ETV), and tenofovir (TDF). These concentrations were 10 times the concentrations in the peripheral blood of each nucleic acid analog. The reason for this is because the concentration of an orally administered drug in the intraportal blood is reported to be 10 times the concentration thereof in the peripheral blood.

### <Measurement of cytokines>

Many types of cytokines in the culture supernatant were measured using a Bio-Plex Human Cytokine Panel (which enables measurement at one time). Furthermore, IL-10, IL-12, and TNF-a were also analyzed by ELISA.

### <Separation of cell fractions>

For the separation of the cytoplasm and the cell membrane, a MEM-PERTMPlus cell membrane protein extraction kit was used, and the protocol indicated by the product was followed. Briefly speaking, the collected cell was washed and then reacted with a buffer (to perforate the cell membrane) at 4°C for 10 minutes. After centrifuging at 16,000 g for 15 minutes, the supernatant was collected in a new tube (cytoplasmic component). The precipitate (cell membrane) in the tube in the previous step was washed once with the above-mentioned buffer, and then reacted with a cell membrane melting buffer at 4°C for 30 minutes while occasionally applying vibration. Thereafter, after centrifuging at 16,000 g for 15 minutes, the supernatant was collected in a new tube (cell membrane component). The cytoplasmic and nuclear components were separated using a cytoplasm/nuclear extraction kit. That is, the collected cell was washed, thereafter reacted with a buffer (to perforate the cell membrane) at 4°C for 10 minutes, and thereafter centrifuged at 16,000 g for 15 minutes to collect the supernatant in a new tube (cytoplasmic component). The precipitate (cell membrane + nucleus) in the tube in the previous step was washed once with the above-mentioned buffer, and then reacted with a nucleus melting buffer at 4°C for 30 minutes while occasionally applying vibration. Thereafter, after centrifuging at 16,000 g for 15 minutes, the supernatant was collected in a new tube (nuclear component).

### <Gene silencing by siRNA>

Chemically synthesized nucleotide siRNA including control siRNA was obtained from Santa Cruz (Dallas, TX, USA). A cell was transfected with siRNA using a Nucleofector kit (Lonza, Cologne, Germany).

### <Western blotting>

The cell was reacted in a blue-loading buffer at 4°C for 30 minutes and centrifuged at 16,000 g for 15 minutes to collect the supernatant. A sample having the same amount of protein was flowed on an SDS-PAGE gel and then transferred to the membrane. After reacting the membrane to which the protein was transferred with 5% calf serum albumin with a blocking buffer, a primary antibody was diluted with the same solution and reacted at 4°C overnight. The membrane was washed and then reacted with a secondary antibody for 1 hour, and after further washing, the membrane was reacted with Western Lighting ECL Pro NEL 12001EA to detect the band with ImageQuant LAS 4000.

### <Fluorescent immunochemical staining>

AKT2-PH-EGFP tagged with HA was inserted into a pCSII-EF1α-MCS-IRES2-Blasticidin lentivirus vector. The lentivirus was infected with a HT1080 cell, and the cell was cultured in a DMEM solution containing 5 mg/ml of blasticidin. For observation of the intracellular migration of PH-EGFP, phosphorylated Akt, and Akt-PH-EGFP, after culturing HT1080 cell expressing each of them on a cover glass with DMSO, LAM, ADV, ETV, and TDF for 2 hours, the cell was fixed with 3.7% formalin, and thereafter nuclear staining of phosphorylated AKt (S473) and DAPI were performed to observe under a microscope.

### <Surface plasmon resonance analysis>

The binding affinity of the peptide for the compound was analyzed using a surface plasmon resonance biosensor (Biacore X 100, GE Healthcare). Akt1 or BSA was fixed on the surface of a CM5 sensor chip using an amine coupling kit (GE Healthcare). Compounds at different concentrations, which were diluted with phosphate buffered saline containing 5% DMSO (10 mM phosphate buffer, 2.7 mM KCI, 137 mM NaCl, 0.05% surfactant P20, pH 7.4), were injected into the sensor chip at a flow rate of 20 µL/min at 25°C for 120 seconds, and subsequently a buffer solution not containing the compounds was applied for 180 seconds. Kinetic parameters were determined by subtracting the bulk effect of DMSO using a reference flow cell and analyzing the data using Biacore X100 evaluation software (GE Healthcare).

### <Statistical analysis>

To compare nonparametric categorical data, Pearson's chi-squared test and Fisher's exact test were used. One-way analysis of variance was used for analysis of variance. All tests were performed using an IBM Statistical Package for Social Sciences, Statistical Desktop for Japan, V.19.0 (IBM JAPAN), with a p-value < 0.05 considered statistically significant.

### Regulation of cytokine profile in LPS-stimulated PBMC

To test whether a nucleic acid analog affects LPS-induced cytokine production, ex vivo pretreatment of a PBMC from HV of three persons was performed with a nucleic acid analog for 2 hours, and subsequently stimulation with LPS was performed to analyze cytokine production using BioPlex cytokine assay. Among 15 cytokines tested (granulocyte-colony stimulating factor (G-CSF), IL-12, IL-10, interferon (IFN)-γ, granulocyte-macrophage (GM)-CSF, IL-13, IL-17, IL-1β, IL-2, IL-4, IL-6, IL-7, IL-8, monocyte chemotactic and activating factor (MCAF), and tumor necrosis factor (TNF)-α), production of three LPS-induced cytokines (IL-10, IL-12p70, and TNF-α) was changed. ADV or TDF significantly induced IL-12p70 and TNF-α and inhibited IL-10, while LAM and ETV showed only the same effect as that of DMSO (Fig. 1A).

Another HV-derived PBMC was treated in the same manner, and when IL-12p70, IL-10, and TNF-α were measured using ELISA, the results similar to the results of the BioPlex assay were shown (Fig. 1B).

The regulation of these cytokines was ADV- or TDF-dose dependent (Fig. 1C). Similar patterns were observed using the PBMC from HBV patient with chronic hepatitis (CH; Fig. 1D) or liver cirrhosis (LC; Fig. 1E).

When comparing cytokine production in the PBMC obtained from different stages of HBV infection, no significant differences were observed between HV, an asymptomatic carrier, and a CH patient. The production of all cytokines in the LC patient was lower than those in the HV, asymptomatic carrier, or CH patient (Fig. 1F).

### Synergistic induction of LPS-stimulated IL-12p70 production by using in a combination with IFN-α

Ex vivo treatment of the PBMC was performed with a nucleic acid analog, IFN-α, and LPS, and the levels of IL-10 and IL-12p70 in the supernatant were measured. When combined with ADV or TDF, IFN-α synergistically enhanced LPS-mediated IL-12p70 production (Fig. 2).

### Inhibition of mTOR pathway

A CD14+ monocyte was stimulated with LPS, and phosphorylation of MAPK-related proteins (pp38, pERK, and pMSK-1) was analyzed. These proteins were maximally phosphorylated 30 minutes after LPS stimulation (Fig. 3).

Under these conditions, the nucleic acid analog did not inhibit the phosphorylation of these proteins, whereas U0126 and SB202198 respectively inhibited the phosphorylation of ERK and MSK-1 (Fig. 4B).

An mTOR-related protein was maximally phosphorylated 2 hours after LPS stimulation (Fig. 5).

Under these conditions, ADV and TDF inhibited the phosphorylation of Akt and p70S6K (Fig. 4C). ADV and TDF inhibited the phosphorylation of Akt in a dose-dependent manner (Fig. 4D). ADV and TDF also inhibited the phosphorylation of GSK-3β (Fig. 4E).

### Cytokine regulation by mTOR inhibitor

Rapamycin (Fig. 6A) and wortmannin (Fig. 6B), which are mTOR inhibitors, dose-dependently inhibited LPS-mediated IL-10 production and induced LPS-mediated IL-12p70 production. These results indicate that LPS-mediated cytokine regulation by ADV and TDF is due to inhibition of the mTOR pathway.

### Inhibition of transition of Akt to cell membrane

The phosphorylation of several proteins upstream of Akt, such as phosphatidylinositol-3 kinase and phosphoinositide-dependent kinase 1, was analyzed. ADV and TDF did not inhibit the phosphorylation of these proteins (Fig. 7A).

When a HT1080 cell stably expressing Akt 2 -PH-EGFP was treated with a nucleic acid analog for 2 hours to examine using a confocal microscope, ADV and TDF significantly inhibited the transposition of Akt-PH to the cell membrane. This result was consistent with a decrease in Akt phosphorylation levels in the HT1080 cell (Fig. 7B).

When the binding affinity of Akt for ETV, TDF, or tenofovir monophosphate (TFV) was analyzed using a surface plasmon resonance biosensor, it was found that TFV bound to the Akt protein in a dose-dependent manner (Fig. 7C).

When a PBMC was pretreated for 2 hours with perifosine, which is known to inhibit the transition of Akt to the cell membrane, and then stimulated with LPS, the production levels of IL-10 and IL-12p70 were respectively decreased and increased in a dose-dependent manner (Fig. 7D). Furthermore, perifosine also inhibited phosphorylation of Akt in a dose-dependent manner (Fig. 7E).

### Non-inhibition of upstream molecules of mTOR

A phosphatase and tensin homologue (PTEN), an mTOR complex 2, and a 5'-AMP-activated protein kinase (AMPK) are known to inhibit the mTOR pathway. ADV and TDF did not affect PTEN or pPTEN expression level in a LPS-treated CD14+ monocyte (Fig. 9).

A decrease in ADV- and TDF-induced IL-10 levels was not restored by silencing rictor (Fig. 10).

5-Aminoimidazole-4-carboxamide-1-β-D-ribofuranoside (AICAR), which is an AMPK inducer, inhibited IL-10 production and induced IL-12p70 when incubated with the LPS-stimulated PBMC (Fig. 11A).

However, AICAR enhanced phosphorylation of Akt in a dose-dependent manner (Fig. 11B). IL-10 production in the PBMC using AMPK silencing was not restored (Figs. 11C and 11D).

From these results, it was indicated that PTEN, mTOR complex 2, and AMPK were not involved in the inhibition of IL-10 production by ADV and TDF.

### Antitumor effect of ADV and TDF using liver cancer cell lines and animal models

Whether ETV or TDF showed an antitumor effect was examined by culturing various liver cancer cell lines (HepG2, PLC/PRF/5, Huh7, Hep3B, SKHep-1, HLE) in ETV or TDF for 72 hours, and thereafter measuring the intracellular ATP amount using CellTiter-Glo (Promega). In all of the cell lines, TDF significantly reduced the number of a survived cell in a dose-dependent manner, but this effect was not recognized in ETV (Fig. 12).

After culturing the human liver cancer cell line (HepG2) in LAM, ETV, ADV, and TDF, phosphorylation of Akt in the mTOR pathway and S6K and 4-EBP downstream thereof was analyzed by western blot. Then, the phosphorylation of the above-mentioned proteins was suppressed in ADV and TDF in a dose-dependent manner, but there was no change in LAM and ETV.

Regarding the mTOR-suppressing effect of ETV and TDF, when the phosphorylation of Akt and GSK-3β (S9) downstream thereof was analyzed by western blotting using mouse liver cancer cell lines (MHCF1 and MHCF5) and a mouse colon cancer cell line (CMT-93), the phosphorylation of mTOR-related protein was suppressed in a HepG2 cell, which is a human liver cancer cell line, whereas the effect of TDF was not recognized in the mouse liver cancer cell lines and the mouse colon cancer cell line (data not disclosed).

90 mg/kg of DMSO (negative control), ETV, ADV, and TDF were intraperitoneally administered to 6-week-old immunodeficient mice (NOD-SCID), respectively (5 animals in each of the groups), HepG2 (5 × 10⁶ cells) was subcutaneously administered 1 week after the start of administration, and the subcutaneous tumor diameter was measured 6 weeks thereafter. Each drug was intraperitoneally administered every 3 days from the start to the end of observation. The tumor diameter in the ETV-administered group was not different from that in the DMSO group, but the tumor diameter was suppressed in the ADV-administered group and the TDF-administered group with a statistically significant difference (p < 0.05 or p < 0.005 for the ETV-administered group or DMSO group, data not disclosed). Furthermore, the tumor did not grow at all in one example in the TDF-administered group, showing an extremely strong antitumor effect.

From these results, the antitumor effect in the liver cancer cell line based on the mTOR pathway suppressing effect of ADV and TDF recognized in the monocyte was recognized in both in vitro and in vivo cases. Furthermore, it was thought that the carcinogenesis suppressing effect of ADV and TDF was based on the Akt phosphorylation suppressing effect of ADV and TDF.

## Claims

1. An mTOR inhibitor comprising:
adefovir or tenofovir or a prodrug thereof, or a pharmaceutically acceptable salt thereof.

2. An Akt phosphorylation inhibitor comprising:
adefovir or tenofovir or a prodrug thereof, or a pharmaceutically acceptable salt thereof.

3. An antitumor agent comprising:
adefovir or tenofovir or a prodrug thereof, or a pharmaceutically acceptable salt thereof.

4. The antitumor agent according to claim 3, wherein
a tumor is a tumor not caused by hepatitis.

5. The antitumor agent according to claim 4, wherein
the hepatitis is hepatitis B.

6. The antitumor agent according to any one of claims 3 to 5, wherein
the antitumor agent is administered to a patient not infected with a hepatitis virus.

7. The antitumor agent according to any one of claims 3 to 6, wherein
the antitumor agent is used in combination with another antitumor agent.
